# EUROPEAN PATENT APPLICATION

(11) **EP 0 779 296 A2**
(43) Date of publication of application: **18.06.1997**
(21) Application number: 97100162.3
(22) Date of filing: 21.06.1993
(51) Int. Cl.: C07K 5/06, A23L 1/236, C07C 237/06, C07C 271/22

(54) **D-alpha-aminoalkanoyl-(S)-N-alpha-alkylbenzyl amides useful as intermediates for artificial sweeteners**

(30) Priority: 22.06.1992 US 902310
(62) Divisional of application: 93915349.0
(71) Applicant: THE COCA-COLA COMPANY, Atlanta, Georgia 30312 (US)
(72) Inventor: D'Angelo, Lihong, Decatur, Georgia 30033 (US); Sweeny, James G., N.W., Atlanta, Georgia 30327 (US)
(74) Representative: Abitz, Walter, Dr.-Ing.

(57) **Abstract**

The present invention relates to D-α-aminoalkanoyl-(S)-N-α-alkylbenzyl amides which are useful as intermediates for preparing artifical sweetener compounds and to methods for preparing these intermediates.

## Description

### FIELD OF THE INVENTION

This invention relates to artificial sweetener compounds comprising L-aspartyl-D-α-aminoalkanoyl-(S)-N-α-alkylbenzyl amides.

### BACKGROUND OF THE INVENTION

L-aspartyl-D-alanine-N-alkyl amides, such as disclosed in U.S. Patent No. 4,411,925, are known to be useful as artificial sweeteners: 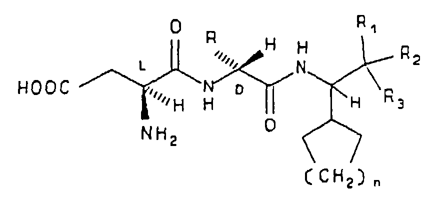 In the series of compounds described by the above structure, the most potent material reported was the L-aspartyl-D-alanine amide of (+/-) t-butyl-cyclopropyl methylamine (R₁ = R₂ = R₃ = CH₃, n = 0; wherein the sweetness potency (SP) was reported to be 1200 times sucrose.) It was furthermore emphasized that the nature of R in the above structure was important for sweetness potency, with R = CH₃ (D-alanine) being especially preferred. In the typical case of the L-aspartyl-D-alanine amides having the structure, 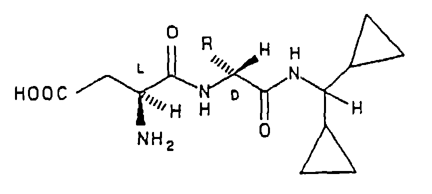 the sweetness potency decreases as R is increased, that is, for R = methyl, SP = 1200, for R = ethyl, SP = 500, and for R = isopropyl, SP = 110.

Zeng et al., J. Agric. Food Chem. 39, 782-85 (1991), disclose L-aspartyl-D-alanine-N-phenyl amides wherein the benzene ring may have methyl-substituents: 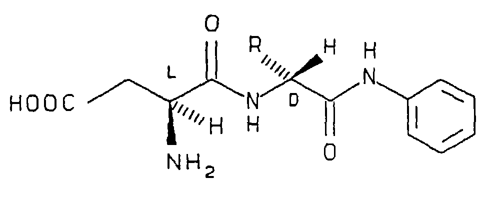 These compounds may be described as aniline amides of L-aspartyl-D-alanine. The individual members of this family of aniline amides were disclosed to have a sweetness potency that was at most 75 times that of sucrose except for the highly substituted 2,6-dimethyl-aniline amide which was disclosed to have a sweetness potency 500 times the sweetness of sucrose. In addition to the relatively low sweetness potency of most of the individual members of this aniline-based series of compounds, these compounds have a potential for toxicity that is known for aniline derivatives.

In addition, Ariyoshi, Bull. Chem. Soc. Japan, 57, 3197 (1984), discloses a series of L-aspartyl-D-alanyl-α-amino acid esters and L-aspartyl-D-valinyl-α-amino acid esters that are tasteless, bitter or of sweetness potency less than 50 times sucrose.

### OBJECTS AND SUMMARY OF THE INVENTION

An object of the present invention is to provide useful chemically stable artificial sweeteners having high sweetness potency using economic, safe and convenient reaction materials.

Another object of the present invention is to provide artificial sweeteners having high heat stability at temperatures typically used for preparing foods.

The present invention provides an artificial sweetener compound comprising an L-aspartyl-D-α-aminoalkanoyl-(S)-α-alkylbenzyl amide having the structure: 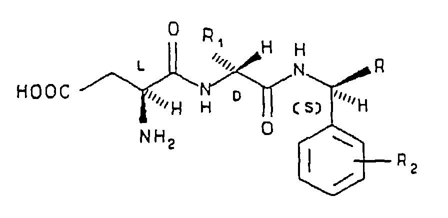 wherein
- R₁ =: H, -CH₃, -CH₂CH₃, -CH₂CH₂CH₃, -CH(CH₃)₂, -C(CH₃)₃, -CH₂OCH₃, -CH₂OH or phenyl
wherein
- R₂ =: H or CH₃;
- R =: 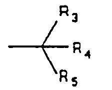 and
- R₃, R₄ and R₅ =: H, -CH₃ or -CH₂CH₃; or
- R =: 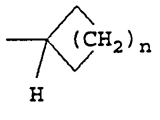
wherein
- n =: 0, 1, 2, 3, 4

### Detailed Description of the Invention

The methods by which the objects, features and advantages of the present invention are achieved will now be described in more detail. These particulars provide a more precise description of the invention for the purpose of enabling one skilled in the art to practice the invention, but without limiting the invention to the specific embodiments described.

The L-aspartyl-D-amino acid amides of the present invention may be conveniently obtained by preparing a dipeptide using any of several known methods for the coupling of amino acids, (e.g., M. Bodansky, Principles of Pentide Synthesis, Berlin, (1984), Springer Verlag), and then coupling the dipeptide with an amine to produce the desired amide.

For example, the method outlined below has been found useful, where R₁ and R₂ are alkyl groups.

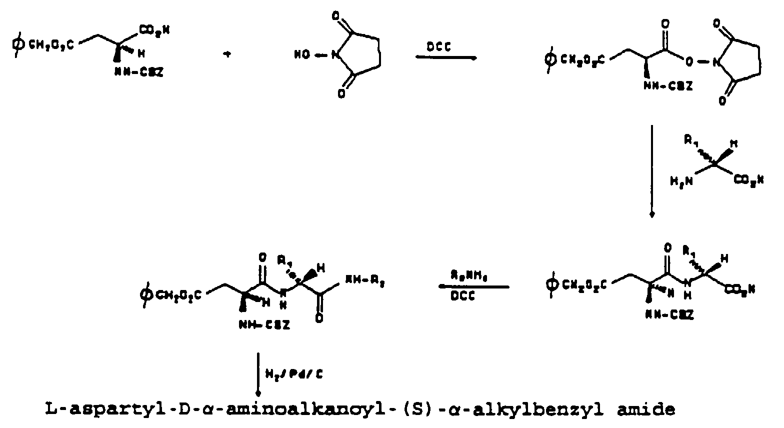

In the first step of the above reaction sequence, a diprotected aspartic acid is condensed with N-hydroxysuccinimide to afford activated aspartyl-N-hydroxy succinimide ester. In this case the diprotected aspartic acid is a β-benzyl-N-carbobenzyloxy ("CBZ") derivative which is commercially available. Condensation of the CBZ-derivative with hydroxysuccimide is achieved by use of dicyclohexylcarbodiimide (DCC) as the coupling agent. The DCC coupling agent is also readily available from known commercial sources.

In the second step, the activated aspartylsuccinimide ester may be reacted with an appropriate D-amino acid in dioxane-water with triethylamine to produce the β-benzyl-N-carbobenzyloxy-L-aspartyl-D-amino acid. The preferred D-amino acids include D-alanine, D-valine, D-α-aminobutyric acid, D-phenylglycine and D-α-aminopentanoic acid. The most preferred D-amino acids of this invention are D-alanine, D-α-amino-butyric acid and D-valine.

In the third step the reaction product of the second step may be activated with DCC and coupled in dioxane with an appropriate amine to afford the β-benzyl-N-carbobenzyloxy-L-aspartyl-D-amino acid amide. The preferred amines of this invention include amines such as α-methylbenzyl amine, α-ethylbenzyl amine, α-isopropylbenzyl amine, α-t-butylbenzyl amine, α-n-propylbenzyl amine, α-phenylbenzyl amine, α-cyclopropyl benzyl amine, and α-iso-butylbenzyl amine. The (S)-enantiomer or a racemic mixture of these amines may be used. Preferably the (S)-enantiomer is used. The most preferred amine of this invention is (S)-α-ethyl-benzyl amine. In the final step the sweetener compound is obtained by deprotection of the product of the third step by catalytic hydrogenation in an alcoholic solvent using Pd/C as catalyst.

While the above method has proven convenient and was used in preparation of all of the compounds described in the examples that follow, other methods may be envisioned which could prove equally advantageous. For example, as described in U.S. 4,411,925, activation of the acid groups of the first and third steps could be achieved using an alkyl chloroformate and a tertiary amine base in place of the DCC. In addition, the use of other protecting groups for the aspartic acid moiety, such as the combination of β-t-butyl ester and N-t-butoxycarbonyl can be envisioned. In this case, deprotection in step 4 would require acid catalysis rather than catalytic hydrogenation.

A second preferred method for preparation of compounds of this invention involves coupling of an N-protected α-amino acid with (S)-alkyl benzylamine (R₂NH₂) as a first step, as shown, wherein R₁ and R₂ are alkyl groups:

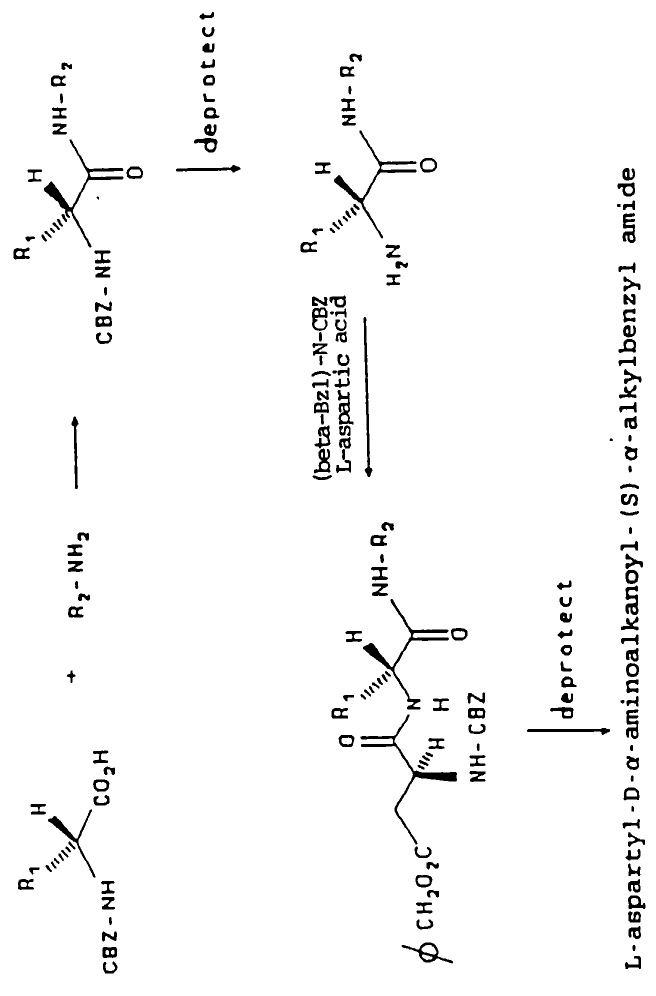

In the second step the protecting group is removed. In the case of a carbobenzyloxy (CBZ) group the protecting group is removed by catalytic hydrogenation. This new amine is then reacted in a third step with N-carbobenzyloxy-β-benzyl-L-aspartic acid and a condensing agent such as DCC to produce a N-carbobenzyloxy-β-benzyl aspartyl-D-amino acid (S)-α-alkylbenzyl amide. The sweetener product is then obtained in a fourth step by catalytic removal of the protecting groups using known methods of hydrogenation over Pd/C catalyst.

As in the first method, other aspartic acid protecting groups such as the t-butyl ester and the N-t-butoxy carbonyl group may be used. Other acid activating reagents such as an alkyl chloroformate and a tertiary amine base may be used in place of DCC.

Still another modification of the second method would involve replacement of the N-carbobenzyloxy-β-benzyl aspartic acid of the third step with an N-protected aspartic anhydride such as the N-carbobenzyloxy- or the N-CHO analogues. Deprotection could then be carried out using hydrogenation over Pd/C in the case of an N-carbobenzyloxy-group or aqueous acid in the case of an N-CHO group. Coupling of amines with aspartic anhydrides usually gives some of the unwanted β-aspartyl amides, but they can be removed from the final product by fractional crystallization. For use of N-carbobenzyloxy-aspartic anhydride, see C.P. Yang and C.S. Su, J. Org. Chem. 51 5186 (1986). For the N-CHO aspartic anhydride, see U.S. Patent No. 3,879,372 or U.S. Patent No. 3,933,781.

The α-alkylbenzylamines used with the present invention are known in the prior art, and may be prepared by reduction of the corresponding ketoxime with sodium in ethanol. The ketoximes may be obtained from the corresponding ketones, which are commercially available. The amines used in the examples had boiling points corresponding to literature values and ¹H and ¹³C NMR spectra consistent with their assigned structures.

Except for the case of the (R)-α- and (S)-α-methylbenzylamines which were purchased as such, the α-alkylbenzyl amines were prepared as racemic mixtures. One of the synthetic amines, the α-ethylbenzylamine, was resolved and resulted in a showing that predominantly the (S)-isomer invokes sweetness. Resolution was achieved by five recrystallizations of the L-(+)-tartaric acid salt of the racemic amine from 95% ethanol.

The other materials, the N-carbobenzyloxy-β-benzyl aspartic acid, the hydroxysuccinimide, the dicyclohexylcarbodiimide (DCC), and the D-amino acids, including D-alanine, D-valine, D-α-aminobutyric acid, D-phenylglycine acid and D-α-aminopentanoic acid are all readily available commercially.

The results of the sweetness potency measurements for the disclosed sweeteners of this invention are summarized in Tables 1-4. The sweetness potency was determined by having four tasters compare the sweetness of various dilutions of the test compound with a 200 ppm equivalent solution of aspartame, wherein the sweetness of aspartame was taken to be 180 times sucrose.

For the L-aspartyl-D-α-aminoalkanoyl-(S)-N-α-alkylbenzyl amides, the presence of the benzyl group, which is novel in itself, produces a high sweetness potency for the various analogues. For example, the results in Table 1 show an increased sweetnesss potency for the S-enantiomer over the R-enantiomer and a high sweetness potency for the (R,S) mixtures of ethyl-, propyl- and t-butyl- α-alkylbenzyl substituents as well as for the compound having an additional phenyl group at the α-benzyl position. Furthermore, contrary to the compounds of U.S. Patent No. 4,411,925, the data presented in Table 2 generally indicate that increasing the size of the R₁ group of the chemical structure shown in Table 2 results in an increase in the sweetness potency. However, the n-propyl-group derivative was found to be less sweet than the methyl-, ethyl- or isopropyl-group derivatives, showing that there are limits on the size of R₁.

The combination of the highest potency of D-amino acid from Table 2, valine, with the highest potency of α-alkylbenzyl substituent from Table 1, (S)-α-ethylbenzyl, might be expected to produce a compound having the highest overall sweetness potency. Indeed, Table 3 shows that L-aspartyl-D-valine-(S)-N-α-ethylbenzyl amide has a sweetness potency of 1500 times sucrose. However, Table 3 shows an unexpectedly higher sweetness potency of 2500 times sucrose for L-aspartyl-D-α-aminobutyric-acid-(S)-N-α-alkylbenzyl amide. The results in Table 3, thus, indicate L-aspartyl-D-α-aminobutyric-acid-(S)-N-α-alkylbenzyl amide and L-aspartyl-D-valine-(S)-N-α-ethylbenzyl amide to be the most preferred sweeteners of the present invention.

The results in Table 4 show that methylation of the aromatic ring of the benzyl group produces a decrease in sweetness potency for each of the compounds shown.

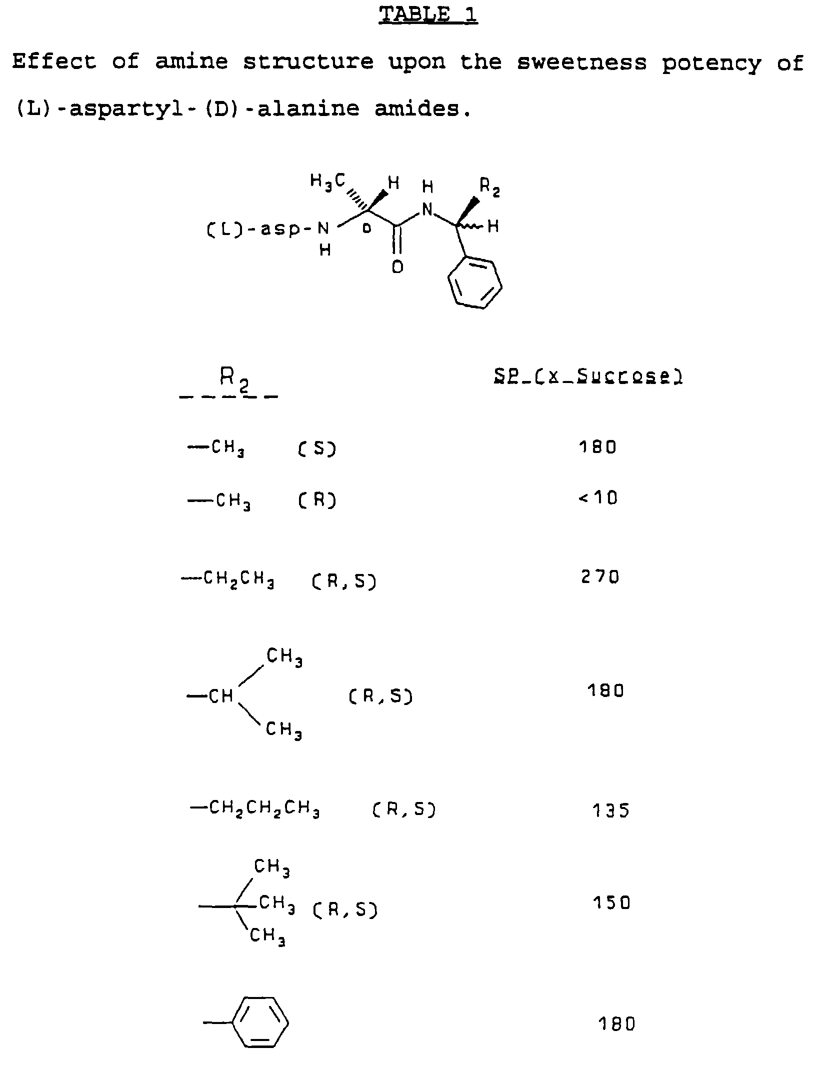

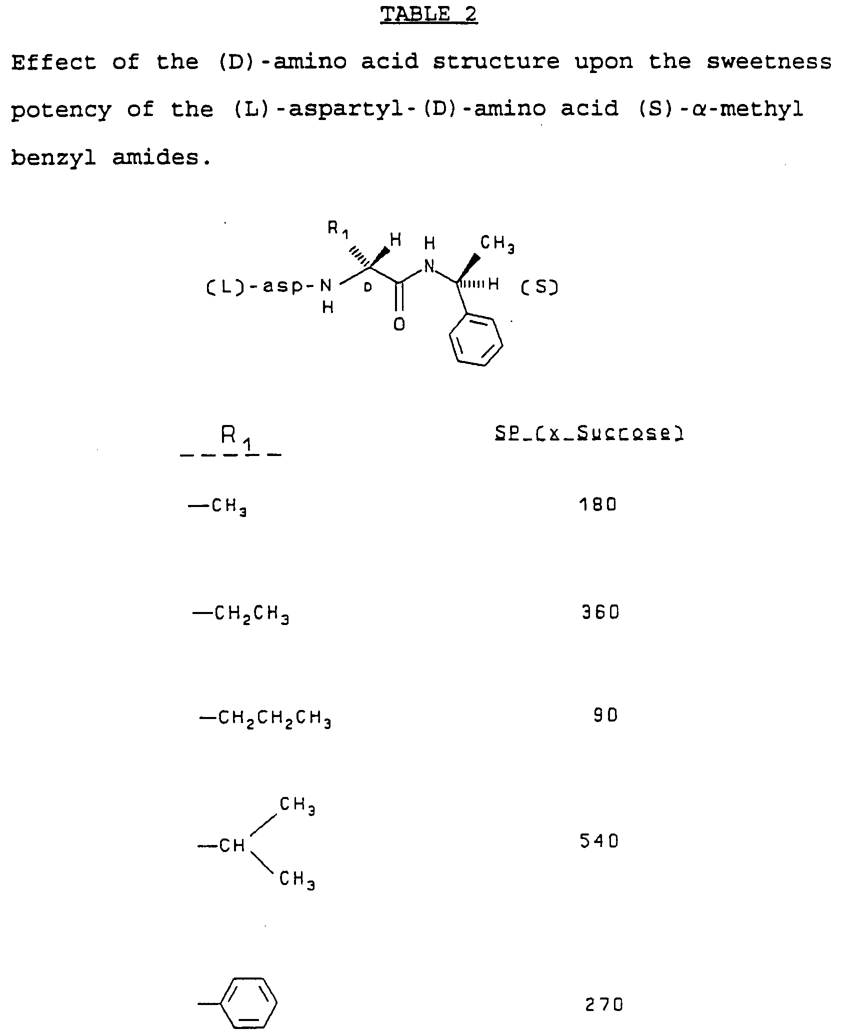

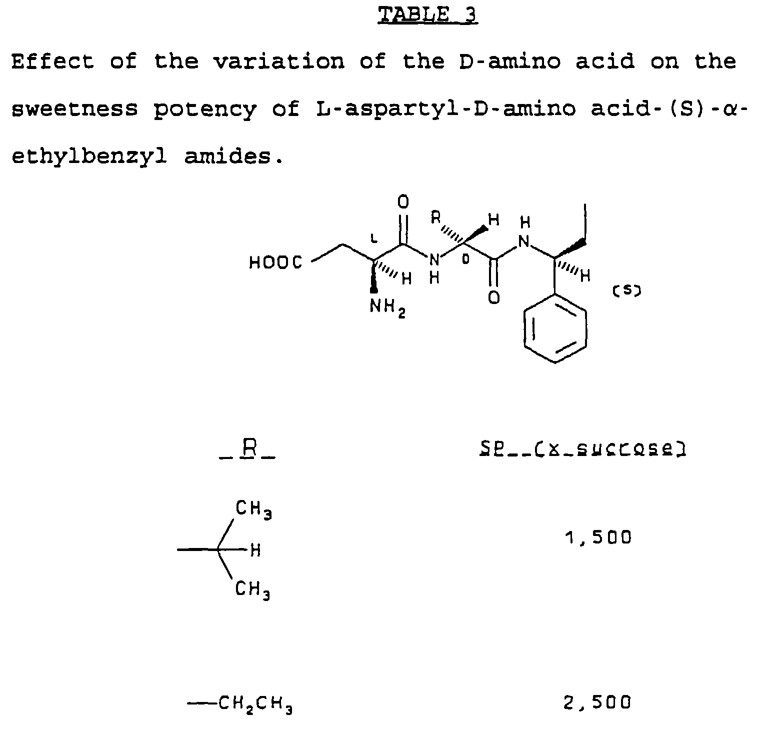

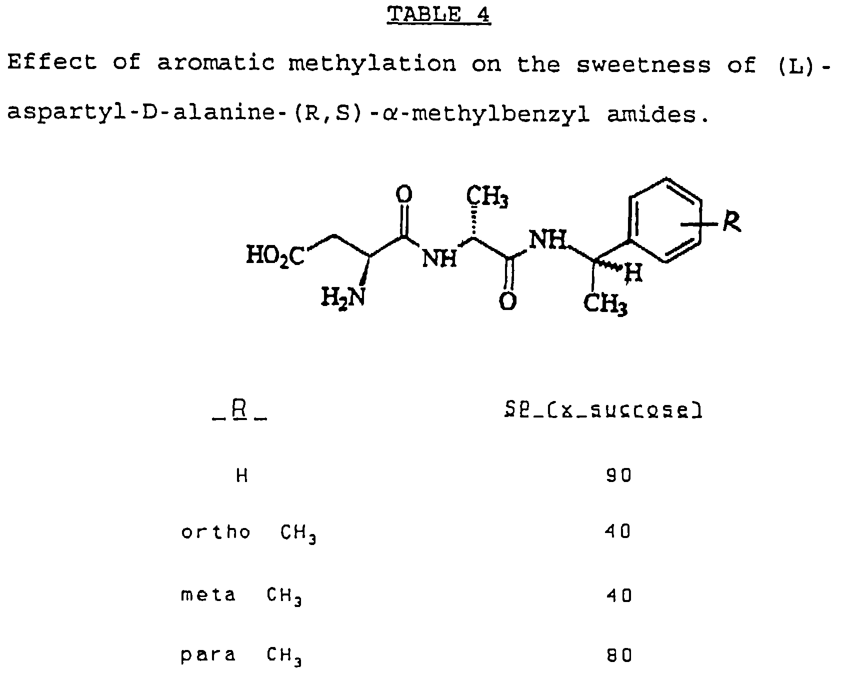

The sweetener compounds and the physiologically acceptable salts thereof of the present invention provide advantages as sweetening agents in view of their high sweetness potency, their physical form and stability. They are, ordinarily, crystalline, non-hygroscopic, water soluble solids. They are characterized by possessing a sweet taste, devoid of undesirable harsh or bitter flavor qualities at ordinary use levels.

The compounds of the invention can be prepared in a variety of forms suitable for utilization as sweetening agents. Typical forms that can be employed are solids, such as powders, tablets, granules and dragees, and livid forms, such as solutions, suspensions, syrups, emulsions, as well as other commonly employed forms that are particularly suited for combination with edible materials. These forms can be comprised of the compounds of the present invention, or of their physiologically acceptable salts, either apart or in association with non-toxic sweetening agent carriers, e.g., non-toxic substances commonly employed in association with sweetening agents. Such suitable carriers include liquids such as water, ethanol, glycerol, corn oil, peanut oil, soybean oil, sesame oil, propylene glycol, corn syrup, maple syrup and liquid paraffin, and solids such as sorbitol, citric acid, lactose, cellulose, starch, dextrin, modified starches, polysaccharides such as polydextrose (see, e.g. U.S. Pat. No. 3,766,165 and 3,876,794), calcium phosphate (mono-, di- or tri-basic) and calcium sulfate.

The sweeteners of this invention may be used to provide desirable properties of sweetness in any orally ingestible product. Examples of specifically ingestible materials include: fruits, vegetables, juices, meat products such as ham, bacon and sausage; egg products, fruit concentrates, gelatins and gelatin-like products such as jams, jellies, preserves, and the like; milk products such as ice cream, sour cream and sherbet; icings, syrups including molasses; corn, wheat, rye, soybean, oat, rice and barley products, nut meats and nut products, beverages such as coffee, tea, carbonated and non-carbonated soft drinks, beers, wines and liquors; confections such as candy and fruit flavored drops, condiments such as herbs, spices and seasonings, flavor enhancers such as monosodium glutamate and chewing gum. The sweeteners may also be useful in prepared packaged products such as dietetic sweeteners, liquid sweeteners, granulated flavor mixes which upon reconstitution with water provide non-carbonated drinks, instant pudding mixes, instant coffee and tea, coffee whiteners, malted milk mixes, pet foods, livestock feed, tobacco and personal care products such as mouth washes and toothpaste as well as proprietary and non-proprietary pharmaceutical preparations and other products of the food, pharmaceutical and sundry industries. Because of their high heat stability at pH 7, these sweeteners are adept for baking applications such as powdered baking mixes for the preparation of breads, cookies, cakes, pancakes, donuts and the like. Especially preferred sweetened edible compositions are carbonated beverages containing one or more of the subject sweeteners. The sweeteners could also be used in frozen desserts, chewing gum, dentifrices, medications or any other orally ingestible substance.

The sweeteners of this invention may also be blended with other sweeteners known to the art, such as, for example, sucrose, fructose and other polyols, as well as other high potency non-nutritive sweeteners including but not limited to saccharin, cyclamate, aspartame, acesulfame-K, alitame, sucralose, stevioside and the like, which are useful for sweetening edible materials. Especially useful are the blends of the sweeteners of this invention and saccharin or physiologically acceptable salts thereof. Examples of saccharin salts include the sodium, potassium, calcium and ammonium salts. Examples of the sweeteners of this invention also include their sulfates, malates, hydrochlorides, carbonates, phosphates, citrates, benzoates and the like. In blends with saccharin the compounds of this invention may reduce or completely mask the well known, undesirable bitter aftertaste of the saccharin.

The invention is further illustrated by the following examples.

### EXAMPLES

### Example 1. N-benzyloxycarbonyl-β-benzyl-L-aspartyl-D-alanine

This example describes the preparation of the protected dipeptide utilized in subsequent examples.

A mixture of 5.0 g N-benzyloxycarbonyl-β-benzyl-L-aspartic acid (14 mmoles), 100 ml tetrahydrofuran, 2.88 g (14 mmoles) dicyclohexylcarbodiimide and 1.61 g (14 mmoles) N-hydroxysuccinimide were stirred at room temperature overnight. The solution was then filtered and the filtrate evaporated to give a thick oil. To this oil was added 80 ml dioxane followed by a solution of 1.5 g (16.6 mmoles) D-alanine, 10 ml dioxane, 20 ml H₂O and 1.85 ml (1.34 g, 13.3 mmoles) triethylamine.

The mixture was stirred again at room temperature overnight. The solution was filtered and the filtrate concentrated to approximately 25 ml. The residue was diluted with 100 ml H₂O, acidified to pH 2.0 with 10% H₃PO₄ and extracted twice with 100 ml ethyl acetate. The combined ethyl acetate layers were backwashed with 100 ml H₂O and 50 ml brine. After drying over Na₂SO₄ the ethyl acetate layer was evaporated to give a white solid. This was crystallized from ethyl acetate-hexane to give 4.2 g, mp 165-67°C. A second crop of 303 mg was obtained at 5°C from the mother liquors to give a total yield of 4.5 g (75%). The literature (U.S. Patent No. 4,411,925) gives mp 158-59°C.

N-carbobenzyloxy, β-benzyl-L-aspartyl-D-valine (57%, mp 93-96°C); N-carbobenzyloxy-β-benzyl-L-aspartyl-D-α-amino butyric acid (57%, mp 151-53°C) [U.S. Patent No. 4,571,345, mp 150-52°C], N-carbobenzyloxy-β-benzyl-L-aspartyl-D-phenylglycine (54%, mp. 64-67°C) and N-carbobenzyloxy-β-benzyl-L-aspartyl-D-α-aminopentanoic acid (73%, mp 91-95°C) were prepared in a similar procedure substituting equivalent weights of the appropriate D-amino acid for the D-alanine.

### Example 2 Synthesis of L-aspartyl-D-alanine-N-(S)-α-methyl-benzylamide.

(a) In a 50 ml flask was mixed 500 mg (1.17 mmoles) N-benzyloxycarbonyl-β-benzyl-L-aspartyl-D-alanine, 0.16 ml (150 mg., 1.24 mmoles) (S)-α-methyl-benzylamine, 241 mg (1.17 mmoles) dicyclohexylcarbodiimide, 210 mg (1.17 mmoles) N-hydroxy-5-norbornene-2,3-dicarboximide and 25 ml dioxane. The solution was stirred at room temperature overnight and filtered. The filtrate was then evaporated to a solid. This was dissolved in 50 ml ethyl acetate, washed twice with 30 ml, 5% aqueous citric acid, twice with 30 ml 4% aqueous NaHCO₃ and twice with 30 ml brine, dried over MgSO₄ and evaporated to give 0.66 g white solid. This was recrystallized from ethyl acetate/hexane to give 0.55 g (1.04 mmoles, 89%) white crystals, mp 168-70°C.
   ¹H NMR (200 MHz, CDCl₃), δ: 7.27-7.33 (m,Ar-H, 15H), 7.2 (d, -N-H, 1H), 7.0 (d, -NH, 1H), 6.0 (d, -NH, 1H), 5.07 (s, -O-CH₂-, 2H), 5.05 (s, - OCH₂-, 2H), 4.4-4.6 (m, N-CH-, 3H), 2.65-3.1 (dd, -CH₂-, 2H), 1.4 (d, C-CH₃, 3H), 1.3 (d,C-CH₃, 3H)
   ¹³C NMR (50 MHz, CDCl₃), δ: 173.6 (-COO-), 173.0 (-CON)-, 172.6 (-CON), 158.0 (-OCON-), 145.6 (-Ar-), 137.9 (-Ar), 137.3 (-Ar), 130.2-130.7 (m, -Ar), 69.3, (-O-CH₂-), 68.8 (-CH₂-O), 53.4 (Ar-CH-N), 51.1 (-CH-N), 50.7 (-CH-N), 38.1 (-CH₂-), 23.8 (-CH₃), 19.5 (-CH₃)
(b) L-aspartyl-D-alanine-N-(S)-α-methyl-benzyl amide
   0.55 g (1.04 mmoles) N-benzyloxycarbonyl-β-benzyl-L-aspartyl-D-alanine-(S)-α-methyl-benzyl amide was dissolved in 50 ml methanol. To this was added 0.06 g 10% Pd on activated carbon. The mixture was hydrogenated at 40 psi overnight. The catalyst was removed by filtration through a Celite® filter material (available from Celite Corporation, Lompoc, CA) and the filtrate was evaporated to give 0.43 g solid. This was then dissolved in 200 ml water and filtered to remove a small amount of the dicyclohexyl-urea that was carried over. The filtrate was freeze-dried to give 0.26 g (0.85 mmoles, 82%) white solid. mp 194-96°C. [α]_{D} = +41.5° (13.0 mg in 1.00 ml methanol).
   ¹H NMR (200 MHz, CD₃OD), δ: 7.26-7.4 (m, -Ar-H, 5H), 5.00 (m, N-CH-, 1H), 4.37-4.42 (m, N-CH-, 1H), 3.95-4.1 (m, N-CH-, 1H), 2.60-2.71 (m, - CH₂-, 2H), 1.44 (d, -CH₃, 3H), 1.38 (d, -CH₃,, 3H).
   ¹³C NMR (50 MHz, D₂O) , δ: 180.5 (CO), 178.7 (CO), 173.8 (CO), 148.0 (Ar), 133.4, 132.0, 130.4 (Ar), 54.9, 54.4, 53.9 (CHN) 41.6 (CH₂CO), 25.5 (CH₃) and 21.1 (CCH₃).

   Sweetness - 180 times sucrose. (The sweetness potency was determined by comparison, using well known methods, against 200 ppm aspartame solution, adopting for aspartame the sweetness potency value of 180 times sucrose.)

### Example 3. Synthesis of L-aspartyl-D-α-aminobutyric-acid N-(S)-α-methylbenzyl amide.

Following the procedure of Example 2, L-aspartyl-D-α-aminobutyric-acid-N-(S)-α-methylbenzyl amide was synthesized using N-carbobenzyloxy-β-benzyl-L-aspartyl-D-α-aminobutyric acid in place of the N-carbobenzyloxy-β-benzyl-L-aspartyl-D-alanine.
Yield of protected intermediate - 56%, mp. 142-46°C
Yield of sweetener - 99%, mp. 176-78°C
Sweetness - 360 times sucrose

### Example 4. Synthesis of L-aspartyl-D-α-aminopentanoic-acid-N-(S)-α-methylbenzyl amide.

Following the procedure of Example 2, L-aspartyl-D-α-aminopentanoic-acid-N-(S)-α-methylbenzyl amide was synthesized using N-carbobenzyloxy-β-benzyl-L-aspartyl-D-α-amino-pentanoic acid in place of the N-carbobenzyloxy-β-benzyl-L-aspartyl-D-alanine.
Yield of protected intermediate - 69%, mp. 158-61°C
Yield of sweetener - 36%, mp. 203-05°C
Sweetness - 90 times sucrose

### Example 5. Synthesis of L-aspartyl-D-valine-N-(S)-α-methylbenzyl amide.

Following the procedure of Example 2, L-aspartyl-D-valine-N-(S)-α-methylbenzyl amide was synthesized using N-carbobenzyloxy-β-benzyl-L-aspartyl-D-valine in place of the N-carbobenzyloxy-β-benzyl-L-aspartyl-D-alanine.
Yield of protected intermediate - 71%, mp. 178-80°C
Yield of sweetener - 45%, mp. 235°C (dec.)
Sweetness - 540 times sucrose

### Example 6. Synthesis of L-aspartyl-D-phenylglycine-N-(S)-α-methylbenzyl amide.

Following the procedure of Example 2, L-aspartyl-D-phenylglycine-N-(S)-α-methylbenzyl amide was synthesized using N-carbobenzyloxy-β-benzyl-L-aspartyl-D-phenylglycine in place of the N-carbobenzyloxy-β-benzyl-L-aspartyl-D-alanine.
Yield of protected intermediate- 46%, mp. 160-64°C
Yield of sweetener - 44%, mp. 211-13°C
Sweetness - 270 times sucrose

### Example 7. Synthesis of L-aspartyl-D-alanine-N-(R)-α-methylbenzyl amide.

Following the procedure of Example 2, L-aspartyl-D-alanine-N-(R)-α-methylbenzyl amide was synthesized using (R)-α-methylbenzyl amine in place of the (S)-isomer.
Yield of protected intermediate - 88%, mp. 167-69°C
Yield of sweetener - 82% mp. 198-200°C
Sweetness - less than 10 times sucrose

### Example 8. Synthesis of L-aspartyl-D-alanine-N-(R,S)-α-ethylbenzyl amide.

Following the procedure of Example 2, L-aspartyl-D-alanine-N-(R,S)-α-ethylbenzyl amide was synthesized using (R,S)-α-ethylbenzyl amine in place of (S)-α-methylbenzyl amine.
Yield of protected intermediate - 86%, mp. 133-34.5°C
Yield of sweetener - 87%, mp. 180-83°C
Sweetness - 270 times sucrose

### Example 9. Synthesis of L-aspartyl-D-alanine-N-(R,S)-α-isopropylbenzyl amide.

Following the procedure of Example 2, L-aspartyl-D-alanine-N-(R,S)-α-isopropylbenzyl amide was synthesized using (R,S)-α-isopropyl-benzylamine in place of (S)-α-methylbenzylamine.
Yield of protected intermediate - 78%, mp. 135-39°C
Yield of sweetener - 90%, mp. 187-91°C
Sweetness - 180 times sucrose
Example 10. Synthesis of L-aspartyl-D-alanine-N-(R,S)-α-t-butylbenzyl amide.

Following the procedure of Example 2, L-aspartyl-D-alanine-N-(R,S)-α-t-butylbenzyl amide was synthesized using (R,S)-α-t-butylbenzylamine in place of (S)-α-methylbenzylamine.
Yield of protected intermediate - 77%, mp. (amorphous)
Yield of sweetener - 90%, mp. 158-63°C
Sweetness - 150 times sucrose

### Example 11. Synthesis of L-aspartyl-D-alanine-N-(R,S)-α-n-propylbenzyl amide.

Following the procedure of Example 2, L-aspartyl-D-alanine-N-(R,S)-α-n-propylbenzyl amide was synthesized using (R,S)-α-n-propylbenzylamine in place of (S)-α-methylbenzyl amine.
Yield of protected intermediate - 84%, mp. 153-55°C
Yield of sweetener - 83% mp. 184-6°C
Sweetness - 90 times sucrose

### Example 12. Synthesis of L-aspartyl-D-alanine-N-α-phenyl benzyl amide.

Following the procedure of Example 2, L-aspartyl-D-alanine-N-α-phenyl benzyl amide was synthesized using α-phenylbenzylamine in place of (S)-α-methylbenzylamine.
Yield of protected intermediate - 83% mp. 165-68°C
Yield of sweetener - 51%, mp. 193-95°C
Sweetness - 180 times sucrose

### Example 13 Synthesis of L-aspartyl-D-alanine-N-α-cyclopropyl benzyl amide.

Following the procedure of Example 2, L-aspartyl-D-alanine-N-α-cyclopropyl benzyl amide was synthesized using (R,S)-α-cyclopropyl benzyl amine in place of (S)-α-methylbenzylamine.
Yield of protected intermediate - 62%, mp. 162-6°C
Yield of sweetener - 93%, mp. 188-90°C
Sweetness - 1,080 times sucrose

### Example 14 Synthesis of L-aspartyl-D-α-aminobutyric acid-N-(S)-α-ethylbenzyl amide.

### (a) N-carbobenzyloxy-β-benzyl-L-aspartyl-D-α-aminobutyric acid-(S)-α-ethyl benzyl amide.

To 250 ml 4% aqueous sodium carbonate was added 3.22 g (11.3 mmoles) (S)-α-ethylbenzyl amine L-(+)-tartrate (mp. 176-79°C). The mixture was extracted twice with 125 ml methylene chloride, the combined methylene chloride extracts dried over Na₂SO₄ and evaporated at <25°C and 20 mm to give a liquid. This was dissolved in 10 ml dioxane and added to a stirred mixture of 5.0 g (11.3 mmoles) N-carbobenzyloxy-β-benzyl-L-aspartyl-D-α-amino butyric acid, 200 ml dioxane, 2.5 g (12.1 mmoles) dicyclohexylcarbodiimide and 1.25 g (7.0 mmoles) N-hydroxy-5-norbornene 2,3-dicarboximide. The mixture was stirred at room temperature overnight, then filtered and the filtrate evaporated to a thick oil. The oil was dissolved in 300 ml chloroform and washed twice with 200 ml 4% aqueous citric acid, three times with 150 ml 4% aqueous NaHCO₃ and with 100 ml H₂O. Drying the chloroform layer over Na₂SO₄ and evaporation of the solvent gave an amorphous solid. Crystallization from ethyl acetate and hexane yielded 5.55 g (9.93 mmoles, 88%) of the protected sweetener, mp. 134-36°C.

### (b) L-aspartyl-D-α-amino-butyric-acid-N-(S)-α-ethylbenzyl amide.

To a solution of 5.25g (9.4 mmoles) of the product of step (a), described above, in 100 ml methanol was added 400 mg of 10% Pd/C catalyst and the mixture hydrogenated at 40 psi H₂ on a Parr shaker for 3 hours at room temperature. The catalyst was removed by filtration through a bed of a Celite® filter material and the filtrate evaporated to give a white solid. This was crystallized from 95% ethanol and acetonitrile to give 1.56 g (4.66 mmoles, 49.6% mp. 197-98°C) of L-aspartyl-D-α-amino-butyric-acid-N-(S)-α-ethylbenzyl amide. A second crop was also obtained: 0.397 g (1.18 mmoles, 12.6%, mp 195-97°C).
1H NMR (200 MHz, CD₃OD) δ:7.2 (m, ArH, 5H): 4.60 (m, -N-CH(R)CO-, 1H), 4.20 (m, -NCH(R)(CO), 1H), 3.94 (m, -CH(R) Ar, 1H), 2.5 (m, CH₂-CO₂H, 2H), 1.55-1.85 (m, CH₂, 4H) and 0.83 (2t, CH₃, 6H).
¹³C NMR (50 MHz, CD₃OD) δ: 175.1, 178.2 and 180.9 (CO), 132.3, 132.6, 134.0, 148.7 (Ar), 61.1 (NHCH(R)Ar), 60.7 (NHCHCO), 56.7 (NCHCO), 42.8 (CH₂C0), 34.7 (CH₂), 31.3 (CH₂), 15.8 (CH₃), and 15.0 (CH₃).
Sweetness - 2500 x Sucrose

### Example 15. Synthesis of L-aspartyl-D-valine-N-(S)-α-ethylbenzyl amide.

Following the procedure of Example 14, L-aspartyl-D-valine-N-(S)-α-ethylbenzyl amide was synthesized using N-carbobenzyloxy-β-benzyl-L-aspartyl-D-valine in place of N-carbobenzyloxy-β-benzyl-L-aspartyl-D-α-aminobutyric acid.
Yield of protected intermediate - 54%, mp. 167-71°C
Yield of sweetener - 42%, mp. 221-22°C
Sweetness - 1500 times sucrose.

### Example 16. Cola Beverage

L-aspartyl-D-α-aminobutyric acid-N-(S)-ethylbenzylamide (0.16 g) is dissolved in 500 ml water and the volume adjusted to one liter. Citric acid (1 g), phosphoric acid (2 g), caramel color (10 g), cola flavoring (10 g) and a benzoate preservative (2 g) are dissolved in the liter solution of sweetener. The resulting cola concentrate is diluted with 3 liters of water to provide a single strength beverage. Carbonation produces a satisfying effervescent carbonated cola drink having a palatable sweetness.

### Example 17. Citrus Beverage

160 mg of L-aspartyl-D-α-aminobutyric-acid-N-(s)-α-ethylbenzylamide is dissolved in 1 liter of water. To this 4.5 g citric acid, 2 g sodium benzoate and 10 g of citrus flavoring are added. The resulting citrus concentrate is diluted with 3 liters of water to provide a single strength beverage. Carbonation as desired gives a satisfactory effervescent beverage having a palatable sweetness.

### Example 18. Dietetic Hard Candy

A hard candy is prepared according to the following formulation and procedure:

| Ingredients | % by weight (approximate) |
|---|---|
| A sweetener | --- |
| FD and C Red #40 (10% aqueous) | 0.05 |
| Cherry Flavor | 0.1 |
| Citric acid | 1.0 |
| Polydextrose* | 70 |
| Water | 30 |

| | |
|---|---|
| *U.s. Pat. No. 3,766,165 | |

The sweetener is an L-aspartyl-D-α-aminoalkanoyl-(S)-N-α-alkylbenzyl amide as disclosed herein, e.g. L-aspartyl-D-α-aminobutyric acid-S-α-ethylbenzyl amide or L-aspartyl-D-valine-S-α-ethylbenzyl amide. The quantity of sweetener added is varied depending on the sweetness potency of the sweetener.

In a small beaker dissolve the sweetener in water, add color, flavor and citric acid and mix well to dissolve. In a separate beaker combine polydextrose and water. Stir while heating to 140°C, then allow to cool to 120°-125°C. Add other ingredients from a small beaker and mix or knead thoroughly. Transfer the material to an oil coated marble slab and allow to cool to 75°-80°C. Extract the material through an oil coated impression roller.

### Example 19. Gelatin Dessert

A gelatin dessert is prepared according to the following composition and procedure.

| Ingredients | % by weight (approximate) |
|---|---|
| Gelatin 225 Bloom | 1.5 |
| Citric acid | 0.36 |
| Sodium citrate | 0.26 |
| Strawberry flavor | 0.06 |
| A sweetener | --- |
| Boiling water | 49 |
| Cold Water | 49 |

The sweetener is an L-aspartyl-D-α-aminoalkanoyl-(S)-N-α-alkylbenzyl amide as disclosed herein, e.g. L-aspartyl-D-α-aminobutyric acid-S-α-ethylbenzyl amide or L-aspartyl-D-valine-S-α-ethylbenzyl amide. The quantity of sweetener added is varied depending on the sweetness potency of the sweetener.

Premix the first five ingredients, add to boiling water and stir to dissolve completely. Add cold water and stir briskly. Transfer to serving dishes and refrigerate until set.

### Example 20. Low Calorie Table Sweetener

Low calorie table sweeteners are prepared according to the following formulations:
A. A powder form of sweetener is prepared by blending the following ingredients.

| Ingredients | % by weight (approximate) |
|---|---|
| A sweetener | --- |
| Crystalline sorbitol | 49.5 |
| Dextrim (dextrose equivalent 10) | 50 |
| Monosodium glutamate | 0.02 |
| Glucomo-delta-lactone | 0.02 |
| Sodium Citrate | 0.02 |

The sweetener is an L-aspartyl-D-α-aminoalkanoyl-(S)-N-α-alkylbenzyl amide as disclosed herein, e.g. L-aspartyl-D-α-aminobutyric acid-S-α-ethylbenzyl amide or L-aspartyl-D-valine-S-α-ethylbenzyl amide. The quantity of sweetener added is varied depending on the sweetness potency of the sweetener.
B. A table sweetener in liquid form is prepared as follows.

| Ingredients | % by weight (approximate) |
|---|---|
| A sweetener | -- |
| Water | 99 |
| Sodium benzoate | 0.10 |

The sweetener is an L-aspartyl-D-α-aminoalkanoyl-(S)-N-α-alkylbenzyl amide as disclosed herein, e.g. L-aspartyl-D-α-aminobutyric acid-S-α-ethylbenzyl amide or L-aspartyl-D-valine-S-α-ethylbenzyl amide. The quantity of sweetener added is varied depending on the sweetness potency of the sweetener.

### Example 21. Frozen Dessert

A vanilla sugarless frozen dessert is prepared according to the following formulation by conventional practice.

| Ingredients | % by weight (approximate) |
|---|---|
| Heavy cream (35% butterfat) | 23 |
| Nonfat milk solids | 10 |
| Mono- and diglyceride emulsifier | 0.25 |
| Polydextrose* | 11 |
| Water | 54 |
| A sweetener | 0.06 |
| Gelatin (225 Bloom) | 0.5 |

| | |
|---|---|
| *U.S. Pat. No. 3,766,165 | |

The sweetener is an L-aspartyl-D-α-aminoalkanoyl-(S)-N-α-alkylbenzyl amide as disclosed herein, e.g. L-aspartyl-D-α-aminobutyric acid-S-α-ethylbenzyl amide or L-aspartyl-D-valine-S-α-ethylbenzyl amide. The quantity of sweetener added is varied depending on the sweetness potency of the sweetener.

### Example 22. Canned Pears

Fresh Pears are washed, peeled, cored, sliced into pieces and immersed in an aqueous solution containing 0.05% by weight of ascorbic acid. The sliced fruit is packed into screw-cap jars and the jars filled with a syrup containing the following ingredients:

| Ingredients | % by weight (approximate) |
|---|---|
| Sorbitol | 25 |
| A sweetener | -- |
| Citric acid | 0.12 |
| Water | 75 |

The sweetener is an L-aspartyl-D-α-aminoalkanoyl-(S)-N-α-alkylbenzyl amide as disclosed herein, e.g. L-aspartyl-D-α-aminobutyric acid-S-α-ethylbenzyl amide or L-aspartyl-D-valine-S-α-ethylbenzyl amide. The quantity of sweetener added is varied depending on the sweetness potency of the sweetener.

The jars are capped loosely and placed in an autoclave containing hot water and processed at 100°C for 45 minutes. The jars are removed, immediately sealed by tightening the caps and allowed to cool.

### Example 23. Powder Beverage Concentrate

| Ingredients | % by weight (approximate) |
|---|---|
| Citric acid | 32 |
| Sodium citrate | 5 |
| Strawberry flavor | 58 |
| Strawberry FD and C color | 0.5 |
| A sweetener | --- |
| Carboxymethyl cellulose | 2.4 |

The sweetener is an L-aspartyl-D-α-aminoalkanoyl-(S)-N-α-alkylbenzyl amide as disclosed herein, e.g. L-aspartyl-D-α-aminobutyric acid-S-α-ethylbenzyl amide or L-aspartyl-D-valine-S-α-ethylbenzyl amide. The quantity of sweetener added is varied depending on the sweetness potency of the sweetener.

Combine all ingredients in a blender and blend until homogeneous. For use, 1.73 g. of powder beverage concentrate is dissolved in 4 fluid ounces (118 ml.) of water.

### Example 24. Baked Cake

A vanilla cake may be prepared employing the following recipe:

| Ingredients | % by weight (approximate) |
|---|---|
| Emulsified shortening | 7.9 |
| Water | 9.9 |
| Eggs | 11.3 |
| Sodium bicarbonate | 0.5 |
| Vanilla extract, single fold | 0.1 |
| Glucono-delta-lactone | 0.8 |
| Polydextrose*, 70% aqueous solution | 39.5 |
| Nonfat dry milk | 1.2 |
| Cake flour | 27.6 |
| Whole milk powder | 0.4 |
| Wheat starch | 0.7 |
| A sweetener | --- |
| U.S. Pat. No. 3,766,165 | |

The sweetener is an L-aspartyl-D-α-aminoalkanoyl-(S)-N-α-alkylbenzyl amide as disclosed herein, e.g. L-aspartyl-D-α-aminobutyric acid-S-α-ethylbenzyl amide or L-aspartyl-D-valine-S-α-ethylbenzyl amide. The quantity of sweetener added is varied depending on the sweetness potency of the sweetener.

Combine nonfat dry milk, whole milk powder, polydextrose solution and emulsified shortening. Mix at low speed until creamy and smooth (about 3 minutes), add eggs and beat until a homogeneous creamy mix is obtained. Dissolve sweetener in water, add to creamy homogenate and mix 2-3 minutes. Add remaining ingredients and mix until creamy and smooth (3-5 minutes). Place 120 g. of batter in small pregreased pan and bake at 350°F. (176°C.) for 30 minutes.

## Claims

1. A compound with the formula: 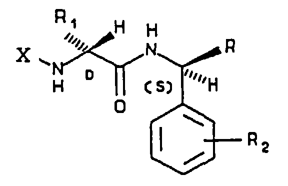 wherein
R₁ = H, -CH₃, -CH₂CH₃, -CH₂CH₂CH₃, -CH(CH₃)₂, -C(CH₃)₃, -CH₂OCH₃, -CH₂OH or phenyl;
wherein
R₂ = H or CH₃;
R = 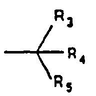 and
R₃, R₄ and R₅ = H, -CH₃ or -CH₂CH₃; or
R = 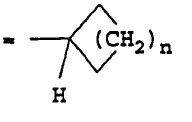
wherein
n = 0, 1, 2, 3, 4 and
X = H or -C(=O)O-CH₂-Ph.

2. A process for preparing a compound of claim 1 by coupling a compound of formula 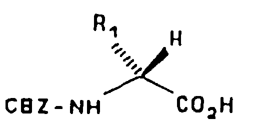 with a compound of formula 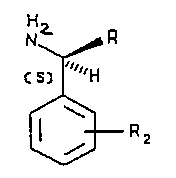 wherein R, R₁ and R₂ are as defined in claim 1 and CBZ is -C(=O)O-CH₂-Ph,
optionally followed by removal of the CBZ group by catalytic hydrogenation.

3. A compound with the formula: 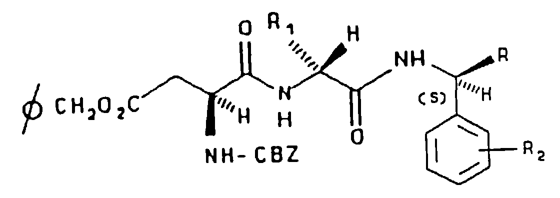 wherein R, R₁ and R₂ are as defined in claim 1, CBZ is as defined in claim 2 and φ is phenyl.

4. A process for preparing a compound of claim 3 by reacting a compound of claim 1, wherein R, R₁ and R₂ are as defined in claim 1 and X is H, with N-carbobenzyloxy-β-benzyl-L-aspartic acid and a condensing agent.

5. Use of a compound of claims 1 or 3 for preparing an artificial sweetener.
